## Europäisches Patentamt

## European Patent Office

## Office européen des brevets

(11) Publication number: **0 140 470**
**B1**

## EUROPEAN PATENT SPECIFICATION

(45) Date of publication of patent specification: **06.09.89**

(51) Int. Cl.⁴: **A 61 F 5/44**

(21) Application number: **84304435.5**

(22) Date of filing: **28.06.84**

(54) **Disposable urinary pad.**

(30) Priority: **23.09.83 US 535193**

(43) Date of publication of application:
**08.05.85 Bulletin 85/19**

(45) Publication of the grant of the patent:
**06.09.89 Bulletin 89/36**

(84) Designated Contracting States:
**AT BE CH DE FR GB IT LI LU NL SE**

(56) References cited:
**EP-A-0 062 495**
**AT-B- 291 429**
**DE-A-2 024 831**
**US-A-2 873 740**
**US-A-3 294 090**
**US-A-3 344 789**
**US-A-3 525 337**
**US-A-3 583 402**
**US-A-3 805 790**
**US-A-4 257 418**
**US-A-4 405 326**

(73) Proprietor: **PERSONAL PRODUCTS COMPANY**
**Van Liew Avenue**
**Milltown New Jersey 08850 (US)**

(72) Inventor: **Holtman, Dennis C.**
**Box 125 RD 5 Old Clinton Road**
**Flemington New Jersey 08822 (US)**

(74) Representative: **Jones, Alan John et al**
**CARPMAELS & RANSFORD 43 Bloomsbury**
**Square**
**London, WC1A 2RA (GB)**

Courier Press, Leamington Spa, England.

# Description

The present invention relates to a dispersable urinary pad having high liquid impact capacity, high liquid retention, and which allows the skin of the weaver to remain dry.

Disposable absorbent products have been known for some time, including such products as disposable diapers, sanitary napkins, wound dressing, bandages and incontinence pads. These products incorporate an absorbent batt which is used to absorb and hold or contain body fluids. Initially in many of these products, especially diapers and sanitary napkins, the absorbent batt comprised what is termed "wadding" or plies of tissue. The wadding was disposed between a liquid-impermeable backing and a liquid-permeable facing and the plies of tissue were used to absorb and, hopefully, contain the liquid within the product. A diaper which utilizes such an absorbent batt is disclosed in US—Re—26,151.

The wadding type of product was replaced, for the most part, by an improved absorbent batt which comprises what is termed "fluffed woodpulp fibers". This absorbent batt comprises a layer of individualized woodpulp fibers with the layer having substantial thickness. A diaper which incorporates such a fluffed woodpulp absorbent batt is described in US—A—2,788,003. This diaper had improved absorbent capacity and somewhat better containment than a diaper using a wadding layer. Also, the fluffed woodpulp layer is quite soft, flexible, and conformable, and, hence, produces an improved diaper over diapers using wadding as the absorbent layer.

Though the fluffed woodpulp absorbent batts have improved capacity, the efficiency with which the capacity is used in a diaper or sanitary napkin is poor. The reason for this is that the fluid to be absorbed is generally deposited in a localized area within the absorbent batt, and the ability of the fluid to move along the plane of the batt is poor. The fluid tends to follow a radial wicking path and consequently moves to the closest edge of the batt where it generally is no longer contained and the product leaks.

US—A—3,017,304 discloses an absorbent product which incorporates in the product a densified papar-like layer. This paper-like layer acts as a wick, i.e. liquid which is placed on the layer tends to move rapidly along the plane of the layer. When incorporated in combination with fluffed woodpulp fiber, the resultant product uses the absorbent capacity of the fluffed woodpulp much more efficiently. Diapers which incorporate this paper-like layer combined with fluffed woodpulp are disclosed and described in US—A—3,612,055 and US—A—3,938,522. This concept of combining wicking ability, or a capillary skin or layer, with fluffed woodpulp fibers has gained wide acceptance in many absorbent products including disposable diapers and sanitary napkins. Even 'though these products make much greater use of the capacity of the absorbent batt, they still do not totally contain the absorbed liquid. It is probable that these products will leak before the full capacity of the batt is used for the absorption or; at the very least, before the entire liquid void by the user is absorbed. This is especially true when pressure is placed on the batt while wet. For example, a baby sitting down on a previously wetted diaper will very often cause the batt to leak.

An incontinent adult faces not only the problems of the infant but many other problems. First, the void of an adult generally is much higher in volume than that of an infant. Second, a bulge under clothing is accepted by society for an infant, but the ambulatory adult with an incontinence problem longs for a product which is not visible through ordinary clothing. Third, the proportions and shape of the legs and torso of the adult differs considerably from those of an infant. Therefore, a mere enlargement of an infant diaper, such as that shown in US—A—4,253,461 is not a satisfactory product.

In both the infant diaper and adult incontinuence product marketplace, a product is needed which has a large storage capacity. For instance, shaped containers have been suggested. However, these containers have been substantinally rigid, do not stay in place, and are quite uncomfortable. A product with a substatnially large storage capacity, with an ability to move liquid away form the void zone, which is disposable, which is confortable, and which does not show through wearing apparel is needed in the marketplace.

A number of years ago, "superabsorbent materials", i.e. materials which will absorb many times their weight of liquid, were developed. Since the development of such materials, attempts to incorporate them in absorbent products such as diapers to enhance the absorption performance of these products have been made. Theoretically, a minimum amount of superabsorbent incorporated in a product would make that product perform as well or better than the prior art products. Perhaps one of the first products to incorporate such a superabsorbent material in a disposable diaper is disclosed in US—A—3,670,731. This patent discloses an absorbent dressing comprising an absorbent layer sandwiched between a permeable facing and an impermeable backing sheet. The absorbent layer contains water-insoluble cross-linked hydrocolloid polymer as the superabsorbent material.

Even though superabsorbent materials have been available for some time, they have not gained wide acceptance in absorbent products such as disposable diapers and sanitary napkins. A primary reason for this lack of acceptance of superabsorbents is failure to develop a product capable of economically utilizing the highly increased absorptive capacity of the superabsorbent material. In order to economically utilize a superabsorbent, the liquid being absorbed must be readily accepted and placed in contact with the superabsorbent material. Furthermore, as the superabsorbent material absorbs liquid, it must be allowed to swell. If the superabsorbent is prevented from swelling, it will cease absorbing liquid. Hence, if the superabsorbent material is to function to absorbent products, such as diapers and sanitary napkins, wherein the liquid to be absorbed is placed in a small void area, the structure of the absorbent layer containing superabsorbent materials must have certain characteristics. Over the years, a number of techniques have been disclosed in an attempt to provide structures which make efficient use of the superabsorbent material. Such products are disclosed in US—A—4,103,062; US—A—4,102,340; and US—A—4,235,237. In addition, methods for incorporating superabsorbents into suitable layers or suitable configurations which can be placed in an absorbent product, are disclosed in US—A—4,186,165; US—A—4,340,057; and US—A—4,364,992. To date, none of these products has met with any substantial commercial success.

The present invention provides an absorbent product which possesses a large storage capacity, which is soft and confortable, which can preferably be designed so as not to be apparent through normal clothing and to utilize a substantial portion of the absorptive capacity of superabsorbent materials. In addition, the absorbent product will contain absorbed liquid even more pressure is placed upon the product during use.

AT—B—0 291 429 discloses an absorbent product comprising a shell containing an absorbent material. The shell may comprise a plastics foam material, such as polyurethane, polyethylene, polystrene or polyvinylchloride foam.

US—A—4 257 418 discloses a holder for multiple use adapted to contain a disposable diaper to form an incontinence pad. The holder is generally boat shaped.

EP—A—0 062 495 discloses an absorbent pad comprising an undulating, unsecured batt made from a sheet of a fibrous absorbent having a water impermeable sheet on one face and being enclosed in a water-permeable wrap.

DE—A—2 024 831 discloses an absorbent urinary pad comprising a shell and a superstructure within the shell. The superstructure comprises polyester fibers in intimate contact with an absorbent medium.

US—A—3 525 337 discloses an absorbent element for sanitary napkins on the like consisting of an accordion-pleated pad formed from a thin layer of absorbent fibers faced on each side with sheets of absorbent cellulose wadding. The pleats are secured in their folded configuration by having the peaks of the folds on one side of the pad attached to an anchoring sheet.

US—A—3 805 790 discloses a disposable sanitary protection product comprising:

US—A—3 805 790 discloses a disposable sanitary protection product comprising:

a liquid impermeable, substantially flexible, moulded shell;

a fibrous superstructure, placed in and substantially filling said shell, which is at least slightly compressible and capable of retaining a void volume of liquid and,

an absorbent medium in intimate contact with at least a portion of said superstructure.

The present invention provides a disposable urinary pad which is characterised in that

the shell has a depth, measured from a line extending across the width at the shell rim in the central portion, of at least 0.5 inch (1.27 cm); and

the superstructure comprises a corrugated fibrous web which is stabilized to retain its corrugations when wet by face-to-face bonding between corrugations.

For example, the shell may be a polyethylene foam shell which is formed from a blown polyethylene foam sheet and subsequently subjected to molding to a thermal process. The shell preferably has a boat-like shape and ranges in thickness from 1/64 inch (0.4 mm) to 1/4 inch (6.4 mm).

The shell has a length which preferably ranges from 4 inch (10.2 cm) to 12 inch (30.5 cm), a width measured from one rim to another across the top space preferably from 2 inch (5.1 cm) to 7 inch (17.8 cm), and a depth of from 0.5 inch (1.5 cm) to 2.5 inch (6.4 cm).

Preferably, the superstructure comprises a fibrous nonwoven web formed of a resilient faiber such as polyester. The web is corrugated and stabilized to prevent the corrugations from separating or flattening when the web is wet and has pressure placed upon it.

The absorbent medium is for example a superabsorbent material, hydrophilic fibers which are loosely compacted or formed into a nonwoven web, wadding, tissue, pest moss or mixture thereof.

Preferably, a liquid permeable fabric or web covers the side of the shell which is open. This cover or facing may be sealed to the rim of the shell thereby entrapping the superstructure and the absorbent medium which has been placed in the shell. If the urinary pad does not have a facing or covering, the superstructure and the absorbent medium are affixed to the shell so as to remain in position even when wet.

The product of the present invention has a high impact capacity, i.e. the product receives a relatively large quantity of liquid and retains it. Furthermore, the product does not leak or spill over. In other words, once the urine enters the pad, the urine remains entrapped within the product. The product also has a high liquid-holding capacity. In addition, the product maintains its surface dry, thereby keeping any moisture away from the skin of the wearer. Still further, the product of the present invention permits air circulation in the region where the product is worn which results in a high degree of comfort.

Some embodiments of the present invention are now described by way of example only, with reference to the accompanying drawings, in which:

Figure 1 is a perspective view of one embodiment of the present invention;

Figure 2 is a perspective view of the parts prior to assembly which provide one embodiment of the present invention;

Figure 3 is a cross-sectional view taken through lines 3—3 of Figure 2; and

Figure 4 is a cross-sectional view, like Figure 3, of still another embodiment of the invention.

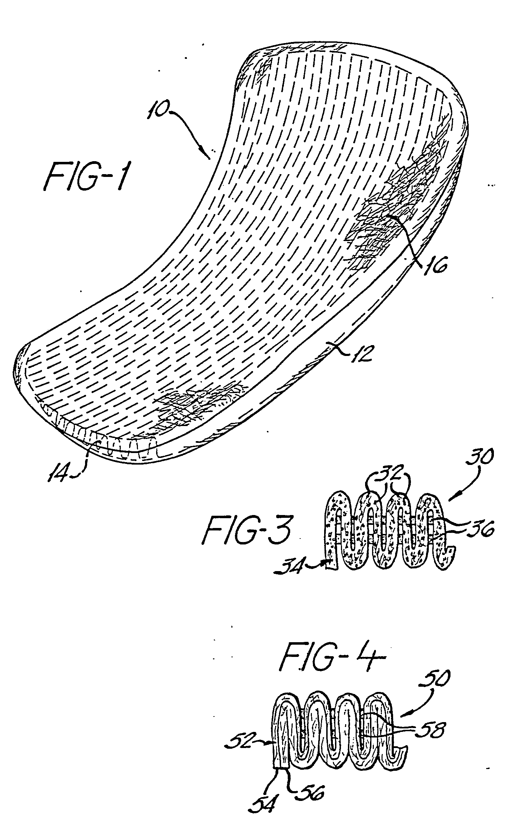

Figure 1 depicts a perspective view of a disposable urinary pad 10 which has a polyethylene foam shell 12 containing a corrugated fibrous web 14 and has a fang 16 sealed 59 the rim of the shell 12.

Figure 2 illustrates the same urinary pad 10 from Figure 1 with the elements separated and showing their relationship. The shell 12 is a polyethylene foam shell which is preformed by a thermal molding process known in the art. The shell is about 0.0625 inch (1.6 mm) thick and is boat-shaped. The top of the shell rims are at least 0.5 inch (1.27 cm) in depth from the bottommost point of the shell. On the underside of the shell 12 are adhesive lines which are applied to provide the securement means for securing the urinary pad to the clothing of the user. These adhesive lines are covered with release strips which when peeled from the adhesive strips leave the adhesive tacky. The superstructure 14 is a corrugated fibrous web which generally is at least 0.5" (1.7 cm) thick and contains about four or five corrugations per inch (2.54 cm). In a specific embodiment, a polyester fibrous web is carded which web has a basis weight of about 25 grams per square meter. The web is corrugated, in other words, transversely folded, by known procedures such as that exemplified in US—A—4,111,733. The web is stabilized by face-to-face bonding so that when the web becomes wet, it does not lose its corrugated configuration. In this embodiment, the absorbent medium (not shown) is affixed to the corrugated web as small particles or film-like partial coverings of the web fibers. The facing 16 is a liquid-permeable, generally hydrophobic fibrous web which may have a typical basis weight of 0.5 oz/yd$^2$ (17 g/m$^2$). The three elements, the shell 12, the absorbent structure 14, and the facing 16, are combined as shown in the drawing, the facing being sealed at its edge to the riom of the shell so as to provide a unitary product.

Figure 3 is a cross-sectional view along line 3—3 of Figure 2 showing a portion of a typical corrugated web. This portion 30 of the web shows the web 34 in a corrugated form wherein superabsorbent 32 has been placed among the fibers of the web. The web has been stabilized by thermal bonding of fusible fibers 36 which are in the blend of fibers forming the web 34.

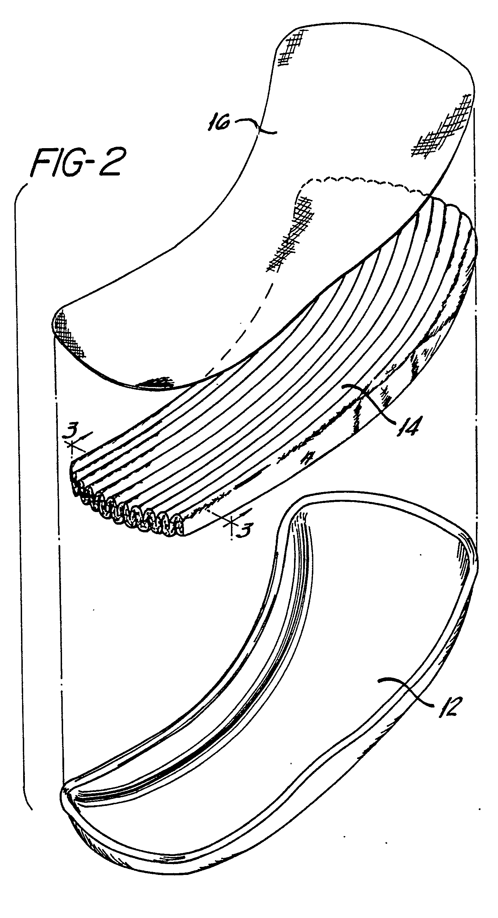

Figure 4 is a cross-sectional view of still another portion 50 of a corrugated web 52. This corrugated web 52 contains two layers 54 and 56. The layer 54 is a fibrous layer which has a lower capillary pressure than the second layer 56. The corrugated web 52 is stabilized by fusible fibers 58. When the web is exposed to a temperature which substantially melts these fibers, the corrugations in the web are partially fused together.

These and other products such as a rectangular incontinence pad, or a smaller urinary pad for a young child or possibly an infant, may be made along the same lines as the products depicted in Figures 1 to 4.

The liquid-impermeable substantially flexible shell is formed from a moldable substance. The substance when molded should preferably provide a liquid-impermeable, substantially flexible shell with a thickness ranging from 0.015625 to 0.25 inch (0.4 mm to 0.64 cm). The shell when deformed should substantially return to its original shape. Substances which provide these cahracteristics and which are moldable, for instance by pressure, or thermal molding, are suitable. Particularly suitable for use in the present invention is a polyethylene-containing foam.

The preferred polyethylene-containing foam shell is prepared by known thermal molding processing. The preferred formulation for forming the polyethylene-containing foam material is identified as Volara®, which is a polyethylene, ethylvinyl acetate blend. The product is manufactured and sold by Voltek, Inc., Lawrence, Masschusetts. Preferably, the formulation is prepared in sheet form at approximately 0.125" (3.2 mm) in thickness. The sheet is subjected to thermal molding at a temperature of about 260°F (127°C) to form the foam shell. The shell is boat-like in shape, but is not limited thereto. The length of the shell preferably ranges from 4 to 12 inch (10.2 to 30.5 cm), with a preferred width from 2 to 7 inch (5.1 to 17.8 cm). The thickness of the shell preferably ranges from 0.015625 to 0.025 inch (0.4 to 6.4 mm). The depth of the shell is measured by extending a line horizontally from one rim to another in the center of the crotch region. The depth is then measured from that line to the base of the foam shell on the longitudinal axis. This depth preferbaly ranges from 0.5 to 2.5 inch (1.27 to 6.7 cm). The foam shell may be made of other suitable compositions, which are soft and flexible and are liquid-impermeable.

The superstructure may be comprised of one or more fibrous webs possibly mixed with resilient fibers which are entangled. A fibrous web generally is formed from synthetic fibers such as polyethylene, polypropylene, polyester, polyamide fibers, bi-component fibers such as those having a polyester core and polyethylene sheath configuration, copolymers thereof, or mixtures thereof. However, cellulosic fibers such as rayon may also be used. The fibers are placed in the web by known means such as by carding to form a web which is then stabilized by face-to-face bonding. Stabilization may be achieved by heat-through bonding, adhesive bonding, point embossing with heat or adhesive (or both), needle punching or use of water jets. The stabilizing process is selected according to the fibers used and the process used to form the web. Other suitable procedures for forming a web include air-laying, wet-laying, spun bonding, laying of melt-blown fibers, spread tow, and other known tech-

niques. A typically suitable web has a dry bulk of at least 10 cc/g and a weight less than 4 oz/yd² (136 g/m²).

The fibrous web is corrugated and stabilized by face-to-face bonding so as to prevent loss of corrugation when the fibrous web becomes wet. Corrugating or transverse folding of the web may be carried out by procedures such as that in US—A—4,111,733. Generally, the web corrugations will range from 3 to 6 or even 8 per inch (2.54 cm) of corrugated web, and the web thickness will be 0.25 to 3 inch (6.4 mm to 7.6 cm), preferably 0.5 to 1.0 inch (1.27 to 2.54 cm) thick. One method of stabilizing the corrugations in the web is accomplished by using an adhesive which may be a latex binder or other known adhesive.

Another method of stabilizing the web is by adding a small portion of fusible fibers to the web fibers before or after the web is made. These fusible fibers have a lower melting point than the remaining fibers and when the corrugated web is subjected to temperature sufficient to melt the fusible fibers, light bonding is provided between the corrugations.

In one specific embodiment, a blend of staple polyester fibers with a minor portion of fusible fibers such as lower melt polyester fibers are carded to form a web. The web is subsequently lightly bonded by passing hot air through the fibers making the fusible fibers tacky so as to stick to each other and the staple fibers to provide the desired degree of integrity to the corrugated web structure.

If the desired thickness is not available in the web, more than one layer of the web may be used but preferably the capillary pressure provided by each web layer increases as the layers are placed away from the facing. In an example with three layers of fibrous web, the top layer, that is the layer closest to the open side of the shell, has the lowest capillary pressure, the said layer has a higher capillary pressure than the first layer but a lower capillary pressure than the third and last layer.

What appears to be only a small difference in capillary pressure is all that is required for one layer to attract and drain liquid from an adjacent layer. The force causing a liquid to enter a cylindrical capillary is expressed by the equation:

$$P = \frac{(2v \cos \theta)}{r}$$

wherein the force is represented by whe capillary pressure and

    P is the capillary pressure,
    v is the surface tension of the liquid,
    θ is the liquid-fiber contact angle, and
    r is the capillary radius.

    With a given liquid, the pressure (capillary force) increases with the cosine of the liquid-fiber contact angle (reacting a maximum where the angle is zero) and also increases with narrower capillary radii so that narrower capillaries will draw liquid from wider ones.

The relative wickability between a first fibrous layer and a second layer is affected by both the relative densities of the layers and the relative wettability of the individual fibers in each layer. The individual fibers of the second layer preferably have substantially smaller liquid fiber contact angles than those of the first fibrous layer overcoming the density difference and providing a significant overall increase in capillary pressure to absorb liquid into the second layer.

The fibers of the second layer of fibers and any subsequent layer of fibers and/or the density of the layer are selected to create a significant difference in capillary pressure from the first fibrous layer.

The second fibrous layer is generally comprised of fibers having a lower liquid-contact angle or wherein the layer is provided with a narrower capillary radii. Examples of such fibers include hydrophilic fibers such as rayon fibers, cellulosic fibers, or peat moss, or mixtures thereof, or acrylic fibers. Cellulosic fibers include wood pulp fibers and cotton linters.

The wood pulp fibers generally are those that are used to form the fluff or fibrous batt layer in conventional absorbent products such as disposable diapers and sanitary napkins. Other cellulosic fibers that might be used are rayon fibers, flax, hemp, jute, ramie and cotton. The fiber, or peat moss, or mixtures thereof are placed in such a way as to form a layer in which the particles are close to one another so as to promote wicking of liquid in the plane of the layer.

The second layer can be preformed and placed next to the first fibrous layer, or the particles (fibers or peat moss or mixtures thereof) can be air-laid, wet-laid, or otherwise combined with the first fibrous layer before transverse folding or corrugating takes place.

The multiple layer structure is then corrugated.

Entangled fibers may be used to provide the superstructure. These fibers should be resilient and have sufficient denier (Tex) to provide the adequate void volume within the foam shell. The fibers may be frictionally entangled or otherwise entangled so as to provide the necessary void volume. Typical of fibers suitable for use are synthetic fibers such as polyethylene, polypropylene, polyester, nylon, bi-component fibers, copolymers thereof and mixtures thereof, cellulosic fibers such as rayon fibers and acrylic fibers.

The superstructure is selected so as to provide sufficient void volume to hold a normal liquid void and impact capacity to receive the liquid rapidly enough to prevent a run off. Also, the superstructure should retain the liquid even under normal pressure such as that provided by the wearer of the pad when sitting down or moving the legs thereby compressing the ordinary pad.

One means of providing an increase in the liquid capacity of the product is the placement of superabsorbent in intimate contact with at least a portion of the superstructure.

The superabsorbent, present either on the fibers or placed in the folds of a corrugated web, or otherwise associated with the void volume portion of the superstructure, is generally a water-insoluble, water-swellable polymeric substance capable of absorbing water in an amount which is at least 10 times the weight of the substance in its dry form. The superabsorbent may be in the form of fibers, spheres, particles, bits of film, globules, webs or film, or may be applied in the form of a liquid monomer solution which is subsequently polymerized. The superabsorbent prepared by polymerization of a monomer solution placed on fibers in a web is most frequently in the form of globules and bits of film-like particles in the web structure.

One type of superabsorbent material provides particles or fibers which may be described chemically as having a backbone of natural or synthetic polymers with hydrophilic groups or polymers containing hydrophilic groups being chemically bonded to the backbone or an intimate mixture therewith. Included in this class of materials are such modified natural and regenerated polymers as polysaccharides, including cellulose, and regenerated cellulose which are modified by being carboxyalkylated, phosphonoalkylated, sulfoalkylated, or phosphorylated to render them highly hydrophilic. Such modified polymers may also be cross-linked to improve their water-insolubility.

These are polysaccharides may also serve, for example, as the backbone on to which other polymer moieties may be bonded by graft copolymerization techniques. Such grafted polysaccharides and their method of manufacture are described in US—A—4,105,033 and may be described as polysaccharide chains having grafted thereon a hydrophilic chain of the general formula:

$$\left[\begin{matrix} (CH_2)_q-CR^1 \\ | \\ C=O \\ | \\ A \end{matrix}\right]_r, \quad \left[\begin{matrix} (CH_2)_p-CR^2 \\ | \\ C=O \\ | \\ B \end{matrix}\right]_s$$

wherein A and B are $-OR^3$, $-O$(alkali metal), $-OHNH_3$ or $-NH_2$, wherein $R^1$, $R^2$, and $R^3$ are hydrogen or alkylene having 1 to 4 or more carbon atoms are wherein r is an integer having a value of 0 to 5000 or more, s is an integer having a value of 0 to 5000 or more, r plus s is at least 500, p is an integer having a value of 0 or 1, and q is an integer having a value of 1 to 4. The preferred hydrophilic chains are hydrolyzed polyacrylonitrile chains and copolymers of polyacrylamide and polysodium acrylate.

In addition to the modified natural and regenerated polymers, the hydrocolloid component may comprise wholly synthetic hydrophilic particles. Examples of those now known in the art are polyacrylonitrile fibers which may be modified by grafting moieties thereon such as polyvinylalcohol chains, polyvinyl alcohol itself, hydrophilic polyurethane, poly(alkyl phosphonates), partially hydrolyzed polyacrylamides (e.g. poly(N-N-dimethylacrylamide), sulfonated polystyrene, or a class of poly(alkyleneoxide). These highly hydrophilic synthetic polymers may be modified by other chemical treatments such as cross-linking or hydrolysis. Further examples known in the art are the non-ionic polymers such as polyoxyethylene, polyoxypropylene, and mixtures thereof which have been suitably cross-linked, either chemically or by irradiation. Still another more recent type is a derivative of isobutylenemalic and acrylate monomers, such as sodium, potassium, ammonium, (or a combination of cations), acrylate, which may be placed on the absorbing layer by spraying or otherwise placing a solution thereon, followed by polymerization and cross-linking, for example by irradiation.

In addition, naturally occurring materials such as gums may be used. Examples of such suitable gums include quar gums, acacia gums and locust beam gums.

The superabsorbent may be placed in the bottom of the shell prior to the placing of the superstructure in the shell or it may be a part of the superstructure. If the superstructure is a fibrous web having substantially uniform density throughout, the superabsorbent is best ppaced between the fibrous web and the inside surface of the shell. Another alternative method of placing superabsorbent on or within a fibrous web, is by spraying a monomer solution on the fibrous web or perhaps even saturating the web with a monomer solution followed by polymerization and cross-linking of the monomer. One typical way to polymerize the monomer is by use of irradiation. This places the superabsorbent substantially evenly throughout the fibrous web and affixes the superabsorbent in such a manner that the superabsorbent globules or particles are within a void volume sufficient to permit them to swell substantially to completion.

The superabsorbent can be placed within the folds provided it is sufficiently associated with the void volume that the swelling of the superabsorbent can occur. If the web is a multiple layer web, it is desirable to associate the superabsorbent with the web having the highest capillary pressure. Another concept in the placement of superabsorbent in a pre-established situation, such as within a moisture-permeable bag such as a "tea-bag", or a pocket. If the superabsorbent is in the form of granules, it may be desirable to moisten the granules and then fix them in place either on the web or in the foam or at the surface of the shell, which will be in contact with the superstructure.

In summation, the fibrous corrugated, stabilized superstructure can be any such structure which has at least a slight degree of compressibility, which allows liquid to enter the structure rapidly, that retains the liquid, and provides collapse resistance so that the liquid is not pressed out of the superstructure.

The product of the present invention does not require a facing or cover but if no facing or covering is used, then it is necessary to secure the superstructure within the shell so that prior to or during use the superstructure does not separate itself form the shell. If, however, it is desirable to one a covering of facing, the covering or facing placed over the open side of the shell is liquid-permeable and is readily sealable to the outer rim of the shell so as to entrap the superstructure in the shell. Suitable coverings or facings include fabrics, nonwoven webs and perforated films. Preferably, the facing is a thermoplastic substance which can be heat sealed to the rim of the liquid-impermeble shell.

The product of the present invention is worn by the wearer in the crotch region, and for simplicity is secured to the underclothing of the wearer. Securement may be effected by adhesive lines or strips on the exterior of the shell or may simply secure itself to the underclothing by means of friction. If the product is to be secured by friction, a material for manufacturing the shell is selected which will provide sufficient friction or a material is coated on the exterior of the shell to provide such friction.

Examples for the preparation of the present invention are as follows.

Example I

A soft flexible shell is formed by thermoforming a polyethylene-ethylvinyl acetate blend foam sheet. The shell has a length 8" (20.3 cm), a width at the widest point of 4.375" (11.1 cm) and a width at the central portion at its narrowest point of 3.75" (9.5 cm). The shell is 0.875" (2.2 cm) deep at the center from a line extending across the center from the edge of each rim of the shell.

The superstructure placed in the shell consists of a two layer fibrous web which has been corrugated. The upper layer (which consists of 50 percent by weight of the web) is a blend of 75 parts of polyester fibers with 25 parts of polyester binder fibers. The average denier (Tex) of the fibers is about 15 (1.7). The second layer (also making up 50% of the web) is 40 parts Orlon® fibers of 1.5 denier (0.17 Tex) blended with 10 parts by weight of the same binder fibers used in the top layer. Each of the layers of the web are carded and one layer is placed upon the other. The two layer web structure is then corrugated and heat set at about 315°F (157°C). The corrugated two layer web 0.75" (1.9 cm) high and has approximately 4.5 folds per inch (2.54 cm) of corrugation. The corrugated web has a weight of about 12 oz/yd² (407 g/m²).

The absorbent medium is a blend of superabsorbent identified as 10Sh manufactured and sold by Mitsubishi Company, Tokyo, Japan, and mineral oil. Four parts of superabsorbent are mixed with one part of mineral oil. This blend is placed on the bottom fiber layer of the two layer web and between the folds to a depth of approximately of 0.25—0.5" (0.64—1.27 cm). The amount of the blend added is approximately 1 gram of the superabsorbent-mineral oil blend per gram of superstructure.

A nonwoven fabric made from bicomponent fibers of polyester core and polyethylene sheath having a weight of about 0.5 oz/yd³ (17 g/m²) is heat sealed to the rim of the shell to provide a facing or covering for the product.

The product is tested by adding 20 cc per second of simulated urine liquid. After the addition of the liquid, the product is left in its receiving position for 5 seconds and then is turned so that the corrugations, if the superstructure is a corrugated product, are vertical. With a discharge of 100 cc (which would occur in 5 seconds), the retention of the product exhibited is 98 percent. With a discharge of 150 cc (in 7 seconds), the retention of the product is 85 percent. With a discharge of 200 cc (over a period of 10 seconds), the retention of the product when turned vertically is 82 percent.

It becomes readily apparent from the above example that the present invention provides a highly satisfactory product for use by incontinent adults who are active people. It is truly surprising that a structure can be provided which will hold at least 80 percent of a 200 cc discharge of urine after merely 5 seconds contact time.

**Claims**

1. A disposable absorbent pad comprising:
a liquid impermeable substantially flexible, moulded shell (12);
a fibrous superstructure (14), placed in and substantially filling said shell (12), which is at least slightly compressible and capable of retaining a void volume of liquid; and
an absorbent medium (32) in intimate contact with at least a portibn of said superstructure,
characterized in that:
the pad is a urinary pad,
the shell (12) has a depth, measured from a line extending across the width at the shell rim in the central portion, of at least 0.5 inch (1.27 cm);
the superstructure (14) comprises a corrugated fibrous web which is stabilized to retain its corrugations when wet by face-to-face bonding between corrugations.

2. The pad of claim 1 wherein the shell (12) comprises polyethylene foam.

3. The pad of claim 1, wherein the shell (12) comprises a thermoformable substance.

4. The pad of any one of claims 1 to 3, wherein the shell (12) has a thickness of 1/64 to 1/4 inch (0.4 to 6.3 mm).

5. The pad of any one of claims 1 to 4, wherein the shell (12) had a depth from 0.5 to 2.5 inch (1.27 to 6.35 cm).

6. The pad of claim 5, wherein the shell (12) has a depth from 0.5 to 1.5 inch (1.27 to 3.81 cm).

7. The pad of any one of claims 1 to 6, wherein the shell (12) has a boat-like shape.

8. The pad of any one of claims 1 to 7, wherein said corrugated fibrous web is a polyester web.

9. The pad of any one of claims 1 to 7, wherein said corrugated fibrous web is a non-woven web of bicomponent fibers of polyester core and polyethylene sheath configuration.

10. The pad of any one of claims 1 to 9, wherein said corrugated fibrous web comprises entangled resilient fibers.

11. The pad of any one of claims 1 to 10 wherein the absorbent medium is loosely compacted wood pulp fibers.

12. The pad of any one of claims 1 to 10, wherein the asborbent medium is a superabsorbent material.

13. The pad of any one of claims 1 to 12, wherein said corrugated web is face-to-face bonded by adhesive.

14. The pad of any one of claims 1 to 12, wherein said corrugated web is face-to-face bonded by thermal bonding of heat fusible fibers contained in the web.

## Patentansprüche

1. Absorbierende Wegwerfeinlage, umfassend:
eine flüssigkeitsundurchlässige, im wesentlichen biegsame geformte Schale (12);
einen faserigen Aufbau (14), der in der Schale (12) angeordnet ist und dieselbe im wesentlichen ausfüllt, wobei der Aufbeu wenigstens geringfügig zusammendrückbar und befähigt ist, ein ausgeschiedenes Flüssigkeitsvolumen zurückzuhalten; und
ein absorbierendes Medium (32), das sich in inniger Berührung mit wenigstens einem Teil des Aufbaues befindet;
dadurch gekennzeichnet, daß;
die Wegwerfeinlage eine Harnaufnahmeeinlage ist;
die Schale (12) eine Tiefe von wenigstens 0,5 Inch (1,27 cm), gemessen von einer Linie aus, die sich quer über die Breitenerstreckung an Schalenrand im Mittelteil erstreckt, aufweist:
und daß der Aufbau (14) eine gewellte Faserbahn umfaßt, welche durch direkte Bindung zwischen den Wellungen stabilisiert ist, um ihre Wellungen in nassen Zustand beizuberhalten.

2. Wegwefeinlage nach Anspruch 1, worin die Schale (12) Polyethylenschaum umfaßt.

3. Wegwerfeinlage nach Anspruch 1, worin die Schale (12) eine unter Warmeeinwinkung verformbare Substanz umfaßt.

4. Wegwerfschale nach einem der Ansprüche 1 bis 3, worn die Schale (12) eine Dicke von 1/64 bis 1/4 inch (0,4 bis 6,3 mm) aufweist.

5. Wegwerfeinlage nach einem der Ansprüche 1 bis 4, worin die Schale (12) eine Tiefe von 0,5 bis 2,5 Inch (1,27 bis 6,35 cm) aufweist.

6. Wegwefeinlage nach Anspruch 5, worin die Schale (12) eine Tiefe von 0,5 bis 1,5 Inch (1,27 bis 3,81 cm) aufweist.

7. Wegwerfeinlage nach einem der Ansprüche 1 bis 6, worin die Schale (12) eine bootähnliche Form hat.

8. Wegwerfeinlage nach einem der Ansprüche 1 bis 7, worin die gewellte Faserbahn eine Polyesterbahn ist.

9. Wegwefeinlage nach einem der Ansprüche 1 bis 7, worin die gewellte Faserbahn eine Faservliesstoffbahn aus Zweikomponentenfasern ist, die einen Polyesterkern und einen Polyethylenmantel aufweisen.

10. Wegwefeinlage nach einem der Ansprüche 1 bis 9, worin die gewellte Faserbahn verwirrte elastische Fasern umfaßt.

11. Wegwerfeinlage nach einem der Ansprüche 1 bis 10, worin das absorbierende Medium aus locker verdichteten Holzzellstofffasern besteht.

12. Wegwerfeinlage nach einem der Ansprüche 1 bis 10, worin das absorbierende Medium ein superabsorbierendes Material ist.

13. Wegwerfeinlage nach einem der Ansprüche 1 bis 12, worin die gewellte Bahn direkte Klebstoffbindungen aufweist.

14. Wegwerfeinlage nach sinem der Ansprüche 1 bis 12, worin die gewellte Bahn aufgrund des thermisches Bindens von wärmverschweißbaren Fasern, die in der Bahn enthalten sind, direkt gebunden ist.

## Revendications

1. Un tampon à jeter absorbant comprenant:
une coquille moulée (12) substantiellement souple, imperméable aux liquides;
une superstructure fibreuse (14), placée à l'intérieur et remplissant substantiellement ladite coquille (12), qui est au moins légèrement compressible et capable de retenir un volume de liquide d'évacuation, et
un millieu absorbant (32) en contact étroit avec au moins une partie de ladite superstructure,
caractérisé en ce que:
le tampon est un tampon pour absorber de l'urine,
la coquille (12) a une profondeur, mesurée à partir du milieu d'une ligne traversant la coquille d'un bord à l'autre en son centre, d'au moins 0,5 pouces (1,27 cm);
la superstructure (14) comprend un tissu fibreux plissé qui est stabilisé par liaison face à face entre les plis, pour retenir ses plis lorsqu'il est mouillé.

2. Le tampon de la revendication 1 dans lequel la coquille (12) comprend de la mousse de polyéthylène.

3. Le tampon de la revendication 1, dans lequel la coquille (12) comprend une substance thermoformable.

4. Le tampon selon l'une des revendications 1 à 3, dans lequel la coquille (12) a une épaisseur de 1/64 à 1/4 pouces (0,4 à 6,3 mm).

5. Le tampon selon l'une des revendications 1 à 4, dans lequel la coquille (12) a une profondeur de 0,5 à 2,5 pouces (1,27 à 6,35 cm).

6. Le tampon de la revendication 5, dans lequel la coquille (12) a une profondeur de 0,5 à 1,5 pouces (1,27 à 3,81 cm).

7. Le tampon selon l'une des revendications 1 à 6, dans lequel la coquille (12) est en forme de bateau.

8. Le tampon selon l'une des revendications 1 à 7, dans lequel ledit tissu fibreux plissé est un tissu de polyester.

9. Le tampon selon l'une des revendications 1 à 7, dans lequel ledit tissu fibreux plissé est un matériau non-tissé de fibres à double composants d'une configuration composée d'un noyau de polyester et d'un revêtement de polyéthylène.

10. Le tampon selon l'une des revendications 1 à 9, dans lequel ledit tissu fibreux plissé comprend des fibres entremêlées élastiques.

11. Le tampon selon l'une des revendications 1 à 10, dans lequel le milieu absorbant est en fibres de pulpe de bois lâchement resserrées.

12. Le tampon selon l'une des revendications 1 à 10, dans lequel le milieu absorbant est un matériau superabsorbant.

13. Le tampon selon l'une des revendications 1 à 12, dans lequel ledit tissu plissé est lié face à face par de l'adhésif.

14. Le tampon selon l'une des revendications 1 à 12, dans lequel ledit tissu plissé est lié face à face par liaison thermique de fibres thermofusibles contenues dans le tissu.

FIG-1

FIG-3

FIG-4

FIG-2

16

14

3

3

12